# EUROPÄISCHE PATENTSCHRIFT

(11) **EP 1 764 121 B1**
(45) Veröffentlichungstag und Bekanntmachung des Hinweises auf die Patenterteilung: **20.02.2008**
(21) Anmeldenummer: 05108503.3
(22) Anmeldetag: 15.09.2005
(51) Int. Cl.: A61M 1/00

(54) **Absaugvorrichtung für das Absaugen von Flüssigkeiten in einem Operationsgebiet**
Suction device for removal of liquids from a surgical field
Dispositif d'aspiration des liquides du champ opératoire

(43) Veröffentlichungstag der Anmeldung: 21.03.2007
(73) Patentinhaber: Cardiosmart AG, 5630 Muri (CH)
(72) Erfinder: Christen, Urs, 5637, Geltwil (CH)
(74) Vertreter: BOVARD AG

(56) Entgegenhaltungen:
- WO-A-98/21094
- US-A- 4 324 243
- US-A- 5 445 613
- US-A1- 2004 133 149
- US-B1- 6 579 257

## Beschreibung

### Technisches Gebiet

Die vorliegende Erfindung betrifft eine Absaugvorrichtung für das Absaugen von Flüssigkeiten in einem Operationsgebiet, mit einem Sauger, wobei an einer Saugerspitze des Saugers ein optischer Sensor angebracht ist und über eine optische Verbindung mit einer Steuereinheit verbunden ist, wobei durch die Steuereinheit und mittels des optischen Sensors detektierbar ist, ob sich die Saugerspitze in der Flüssigkeit oder in der Luft befindet.

### Stand der Technik

Bei verschiedenen Operationen an Patienten wie beispielsweise bei einer Herzoperation sickert Blut aus den Operationswunden und kann ein Operationsgebiet stark überfluten. Bei einer durchschnittlichen Herzoperation von etwa 90 bis 120 Minuten können so bis zu etwa 4 bis 6 Liter Blut anfallen. Durch das Blut kann dem Operateur die Sicht auf den zu korrigierenden Defekt verdeckt werden. Zudem muss dieses Blut entweder durch Fremdblut ersetzt werden oder es muss gesammelt und in den Körper des Patienten zurückgeführt werden. Es ist im Stand der Technik bekannt, Blut, welches in einem Operationsgebiet anfällt, mittels einer Absaugvorrichtung abzusaugen. Bei bekannten für die Herzchirurgie geeigneten Absaugvorrichtungen ist ein Sauger, an dessen freiem Ende eine Saugerspitze mit Absaugöffnungen angeordnet ist, über eine Absaugleitung mit einer Absaugpumpe verbunden. Die Absaugleitung ist beispielsweise als PVC- oder Silikonschlauch ausgebildet, welcher in einen u-förmigen Pumpenkörper der Absaugpumpe eingelegt wird. In einer solchen als Rollenpumpe ausgeführten Absaugpumpe werden zwei gegenüberliegende Rollen mit bis zu 250 U/min über die Absaugleitung geführt, wobei die Absaugleitung an der Stelle, an der sich die Rollen befindet, zusammengedrückt wird. Mit einer solchen Rollenpumpe wird ein Sog erzeugt, welcher ausreicht, um das in einem Operationsgebiet anfallende Blut über die Saugerspitze der Absaugvorrichtung abzusaugen und in ein Reservoir zu leiten. Im Stand der Technik werden Absaugvorrichtungen eingesetzt, bei welchen die Rollenpumpe ständig eingeschaltet bleibt. Sobald jedoch alles Blut abgesaugt ist oder sobald die Saugerspitze aus dem Blut genommen wird, wird durch eine solche Absaugvorrichtung Luft angesaugt und es entsteht ein Blut-Luftgemisch. Ein durch eine solche Absaugvorrichtung abgesaugtes Blut-Luftgemisch stellt jedoch ein besonderes Problem dar, da durch auftretende Scherkräfte und durch den Luftkontakt Schädigungen bei den Blutkörperchen und dem Gerinnungssystem des Blutes auftreten. Um das Absaugen von Luft zu minimieren, kann die Rollenpumpe jeweils von Hand gestartet und wieder gestoppt werden. Allerdings ist ein solches An- und Abschalten in vielen Operationsräumen nicht ohne weiteres durchführbar, da die Person, welche den Sauger bedient, keinen direkten Einblick in das Operationsgebiet hat und deshalb auf den Zuruf des Operateurs angewiesen ist, um die Absaugvorrichtung bei vorhandenem Blut einzuschalten und bei fehlendem Blut wieder auszuschalten. Ein solcher Zuruf des Operateurs führt jedoch zu unerwünschten Stresssituationen bei den beteiligten Personen. In der Veröffentlichung WO 98/21094 wird eine Absaugvorrichtung beschrieben, bei welcher ein optischer Sensor an der Saugerspitze angebracht ist. Mittels dieses Sensors kann Flüssigkeit von Luft unterschieden werden und es ist möglich, die Rollenpumpe bei vorhandener Flüssigkeit zu starten und bei Lufteintritt automatisch zu stoppen. Verwirbelungen von Luft mit dem Blut werden dadurch minimiert und das An- und Abschalten der Absaugvorrichtung muss nicht mehr von Hand gesteuert werden. Ein Nachteil einer solchen Absaugvorrichtung ist es, dass diese als vollständig neue und eigenständige Einheit schlecht in eine bestehende Infrastruktur eines Operationsraums integriert werden kann und deshalb nicht verbreitet eingesetzt wird. Da die automatische Steuerung der Rollenpumpe durch einen Defekt ausfallen kann, muss der Operationsraum zudem über eine zusätzliche Ersatz-Absaugvorrichtung verfügen.

### Offenbarung der Erfindung

Es ist eine Aufgabe der vorliegenden Erfindung, eine neue Absaugvorrichtung vorzuschlagen, welche die Nachteile des Standes der Technik nicht aufweist.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Insbesondere werden diese Ziele durch die Erfindung dadurch erreicht, dass der Sauger über eine Absaugleitung mit einem Reservoir verbunden ist, dass das Reservoir über eine Verbindungsleitung mit einer Unterdruckeinheit verbunden ist, dass an der Absaugleitung eine durch die Steuereinheit steuerbare Schliesseinheit angebracht ist, mit welcher Schliesseinheit die Absaugleitung verschliessbar ist, falls mittels der Steuereinheit detektierbar ist, dass sich die Saugerspitze in der Luft befindet. Über die Verbindungsleitung wird ein Unterdruck der Unterdruckeinheit in das Reservoir geführt, womit infolge des Unterdrucks im Reservoir Flüssigkeit vom Operationsgebiet, falls sich die Saugerspitze in der Flüssigkeit befindet, in das Reservoir absaugbar ist. Die Unterdruckeinheit kann beispielsweise durch ein in Operationsräumen eines Spitals schon angebrachtes Vakuumsystem mit einem handelsüblichen Unterdruckregler oder durch ein autonomes Sauggerät gebildet werden. Als Reservoir, Absaugleitung und Sauger können für den Spitalbedarf handelsübliche Komponenten verwendet werden, wobei der optische Sensor an der Saugerspitze des Saugers angebracht wird und die optische Verbindung zwischen dem optischen Sensor und der Steuereinheit entlang der Absaugleitung geführt wird. Die Steuereinheit und die Schliesseinheit können in einer Box angeordnet sein, wobei die Absaugleitung über die Schliesseinheit der Box und die optische Verbindung an die Steuereinheit der Box geführt werden, und wobei die Steuereinheit und die Schliesseinheit geeignet verbindbar sind. Eine solche Absaugvorrichtung hat den Vorteil, dass diese sehr leicht in eine bestehende Infrastruktur eines Spitals integriert werden kann. Bei einem Defekt an der Steuereinheit oder der Schliesseinheit lässt sich die Absaugvorrichtung zudem durch ein einfaches Austauschen oder Entfernen der Box sehr schnell in eine funktionsfähige Absaugvorrichtung überführen.

In einer Ausführungsvariante ist die optische Verbindung durch mindestens eine optische Lichtleiterfaser ausgeführt und der optische Sensor wird durch einen Abschnitt der mindestens einen Lichtleiterfaser gebildet. Der Abschnitt kann sich beispielsweise auf einen Endabschnitt einer einzelnen Lichtleiterfaser beziehen, wobei dieser Endabschnitt geeignet ausgeformt ist, beispielsweise halbkugelförmig ausgeformt ist. Eine solche Ausführungsvariante hat den Vorteil, dass der optische Sensor sowie die optische Verbindung zwischen dem optischen Sensor und der Steuereinheit sehr robust und kostengünstig herstellbar sind.

In einer weiteren Ausführungsvariante umfasst die Steuereinheit einen optischen Sender und einen optischen Empfänger, wobei mittels einer Kopplungseinheit optische Signale vom optischen Sender in die optische Verbindung eingekoppelt wird und zum optischen Sensor geführt wird, wobei der optische Sensor derart ausgebildet ist, dass mit Luft respektive Flüssigkeit als umgebendes Medium das Licht total reflektiert respektive nur teilweise reflektiert wird und über die optische Verbindung und die Kopplungseinheit an den optischen Empfänger zurückgeleitet wird, und dass die Steuereinheit eine Detektionseinheit umfasst, welche derart ausgebildet ist, dass mittels einer Messung der durch den optischen Empfänger empfangbaren Lichtenergie detektiert wird, ob sich der optische Sensor in der Luft als umgebendes Medium oder in Flüssigkeit als umgebendes Medium befindet. Falls die optische Verbindung durch eine einzelne Lichtleiterfaser gebildet wird, dann können die Einkoppelung sowie die Auskoppelung von Licht in diese sowie aus dieser Lichtleiterfaser insbesondere über einen optischen Koppler wie einen Y-Koppler erfolgen. Eine solche Ausführungsvariante hat insbesondere den Vorteil, dass eine sehr genaue Detektion ermöglicht wird, ob sich der optische Sensor in Luft als umgebendes Medium oder in Flüssigkeit als umgebendes Medium befindet.

In einer anderen Ausführungsvariante besteht die Absaugleitung aus einem deformierbaren Material, wobei die Schliessvorrichtung eine Halterung zur Aufnahme der Absaugleitung und einen Schieber umfasst, und wobei der Schieber so angeordnet ist, dass die Absaugleitung durch den Schieber deformierbar und vollständig verschliessbar ist. Eine solche Ausführungsvariante hat den Vorteil, dass das Verschliessen der Absaugleitung mittels eines Schiebers sehr präzise durchgeführt werden kann.

In einer weiteren Ausführungsvariante ist der Schieber mittels einer von einem Elektromotor ableitbaren Kraft oder mittels einer von einem Magnetantrieb ableitbaren Kraft antreibbar. Eine solche Ausführungsvariante hat den Vorteil, dass die Schliessvorrichtung auf optimale elektromagnetische Verträglichkeit oder auf optimale Schliessgeschwindigkeit bzw. Schliessleistung angepasst werden kann.

In einer anderen Ausführungsvariante umfasst die Halterung zur Aufnahme der Absaugleitung mindestens eine Quetscheinheit, wobei die Absaugleitung in der Halterung durch ein Quetschen der Absaugleitung in der Quetscheinheit wiederentfernbar anbringbar ist. Eine solche Ausführungsvariante hat insbesondere den Vorteil, dass bei einem Defekt der Schliesseinheit oder der Steuereinheit die Absaugleitung sehr rasch freigelegt werden kann und somit die Absaugvorrichtung - zwar ohne eine dem an der Saugerspitze entsprechenden Steuerung - sehr schnell weiterverwendet werden kann und die Box bestehend aus Schliesseinheit und Steuereinheit sehr schnell überbrückt werden kann. Diese Ausführungsvariante hat auch den Vorteil, dass eine erfindungsgemässe Absaugvorrichtung in sehr vielen Spitälern mit einer minimalen Änderung an der Infrastruktur eingesetzt werden kann.

In einer weiteren Ausführungsvariante umfasst die Steuereinheit ein Kalibriermodul, wobei beim Einschalten der Steuereinheit mittels des optischen Senders ein Lichtsignal in die optische Verbindung eingekoppelt wird, wobei mittels des optischen Empfängers die reflektierte Lichtenergie gemessen wird und wobei mittels des Kalibriermoduls die Schliessvorrichtung für die gemessene Lichtenergie derart eingestellt wird, dass die Absaugleitung verschlossen ist. Eine solche Ausführungsvariante hat insbesondere den Vorteil, dass die Absaugvorrichtung bei der Inbetriebnahme sehr schnell in einen einsatzfähigen Zustand gebracht werden kann.

### Kurze Beschreibung der Zeichnungen

Nachfolgend werden Ausführungsvarianten der vorliegenden Erfindung anhand von Beispielen beschrieben. Die Beispiele der Ausführungen werden durch folgende beigelegte Figuren illustriert:
Figur 1 zeigt eine schematische Darstellung einer erfindungsgemässen Absaugvorrichtung.
Figur 2 zeigt ein Blockbild eines optischen Sensors, einer optischen Verbindung, einer Absaugleitung, einer Steuereinheit und einer Schliesseinheit.

### Ausführungsform(en) der Erfindung

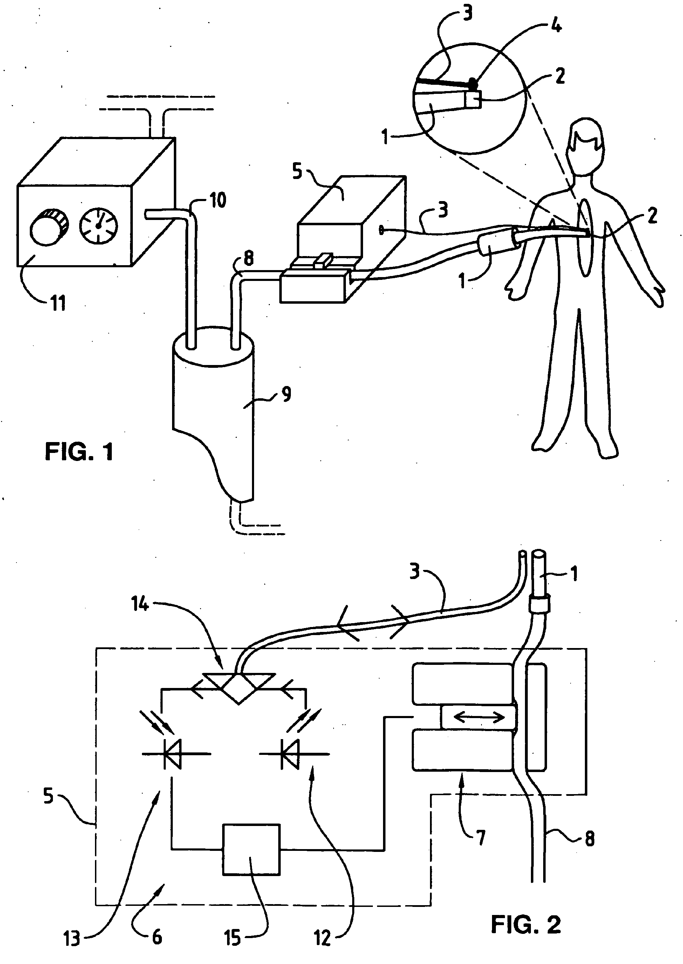

In Figur 1 bezieht sich das Bezugszeichen 1 auf einen Sauger. Der Sauger 1 umfasst beispielsweise einen Saugergriff, eine Saugerlanze und eine Saugerspitze 2. Der Sauger 1 kann beispielsweise aus Kunststoffen wie beispielsweise einem PVC- oder Silikonschlauch aufgebaut sein. Ein solcher Sauger 1 wird von verschiedenen Anbietern hergestellt und kann insbesondere als ein für in einem Operationsgebiet verwendbarer sterilisierter Sauger zur einmaligen Verwendung hergestellt sein. In Figur 1 bezieht sich das Bezugszeichen 3 auf eine optische Verbindung und das Bezugszeichen 4 bezieht sich auf einen optischen Sensor. Der optische Sensor 4 ist an der Saugerspitze 2 angebracht, wobei optische Signale über die optische Verbindung 3 zum optischen Sensor 4 hingeführt und weggeführt werden können. Die optische Verbindung 3 und der optische Sensor 4 beziehen sich beispielsweise auf eine Lichtleiterfaser, beispielsweise auf eine Glasfaserleitung, wobei der optische Sensor 4 z.B. durch ein halbkugelförmig ausgestaltetes Ende der Lichtleiterfaser gebildet wird. Ein solcher halbkugelförmiger Sensor 4 ist dabei derart ausgestaltet, dass optische Signale, also beispielsweise Lichtstrahlen einer bestimmten Wellenlänge, vom halbkugelförmigen Sensor 4 vollständig oder praktisch vollständig reflektiert wird, falls sich der optische Sensor 4 in Luft befindet und dass diese optischen Signale nur teilweise reflektiert werden, falls sich der optische Sensor 4 in Flüssigkeit befindet. Eine solche vollständige oder teilweise Reflexion der Lichtsignale ergibt sich aus den unterschiedlichen Brechungsindizes von beispielsweise Glas, Luft und Flüssigkeit. In Figur 1 und in Figur 2 bezieht sich das Bezugszeichen 5 auf eine Box. Wie in Figur 2 gezeigt, sind in der Box 5 eine Steuereinheit 6 und eine Schliesseinheit 7 angebracht. Die optische Verbindung 3 ist mit der Steuereinheit 6 verbindbar und die Steuereinheit 6 ist mit der Schliesseinheit 7 verbindbar. Die Merkmale und Bestandteile der Box 5, der Steuereinheit 6 und der Schliesseinheit 7 werden später beschrieben. In Figur 1 bezieht sich das Bezugszeichen 8 auf eine Absaugleitung und das Bezugszeichen 9 bezieht sich auf ein Reservoir. Der Sauger 1 ist über die Absaugleitung 8 mit dem Reservoir 9 verbunden. Die Absaugleitung 8 besteht beispielsweise aus einem sterilisierten PVC- oder Silikonschlauch und ist beispielsweise über geeignete Steckverbindungen mit dem Reservoir 9 und dem Sauger 1 verbindbar. In Figur 1 bezieht sich das Bezugszeichen 10 auf eine Verbindungsleitung und das Bezugszeichen 11 bezieht sich auf eine Unterdruckeinheit. Die Unterdruckeinheit 11 kann beispielsweise aus einem an ein in einem Spital vorhandenes Vakuumsystem (in Figur 1 gestrichelt gezeichnet) anschliessbarer Unterdruckregler bestehen, wobei mittels des Vakuumsystems ein Unterdruck erzeugt wird, welcher mittels des Unterdruckreglers auf einen definierten Wert regulierbar ist. Anstelle einer Unterdruckeinheit, welche auf einem vorhandenen Vakuumsystem basiert, kann auch eine autonome Unterdruckeinheit wie beispielsweise ein Sauggerät eingesetzt werden, welches unabhängig von einem spitalinternen Vakuumsystem arbeitet. Die Unterdruckeinheit 11 kann beispielsweise einen einstellbaren Unterdruck von 0 bis - 100 mmHg bei einer Genauigkeit von ± 10 mmHg erzeugen. Über die Verbindungsleitung 10 wird der von der Unterdruckeinheit 11 erzeugte Unterdruck an das Reservoir 9 geführt. Das Reservoir 9 kann beispielsweise ein handelsübliches Kardiotomie-Reservoir sein. Der Aufbau des Reservoirs 9 umfasst beispielsweise Anschlüsse für den Anschluss des Saugers 1, der Unterdruckeinheit 11 und für den Anschluss einer Einheit zum Abführen von Flüssigkeit aus dem Reservoir 9 (in Figur 1 gestrichelt gezeichnet). Durch die Unterdruckeinheit 11 wird im Reservoir 9 ein Unterdruck erzeugt, welcher über die Absaugleitung 8 an den Sauger 1 und an die Saugerspitze 2 geführt wird. Dadurch wird das Medium durch die Saugerspitze 2 angesaugt, wobei Luft angesaugt wird, falls die Saugerspitze 2 in der Luft ist und wobei Flüssigkeit angesaugt wird, falls die Saugerspitze 2 in einer Flüssigkeit ist. Damit lässt sich also beispielsweise Blut, welches in einem Operationsgebiet anfällt, absaugen.

Die Box 5 umfasst eine Steuereinheit 6 und eine Schliesseinheit 7. Box 5 wird auch als SSD-FS1 (Smart-Suction-Device Fibre-Sensor 1) bezeichnet. Selbstverständliche müssen die Steuereinheit 6 und die Schliesseinheit 7 nicht in derselben Box 5 angeordnet sein, sondern können durchaus auch als getrennte Einheiten angeordnet sein, welche über eine Steuerverbindung verbindbar sind. Die Steuereinheit umfasst einen optischen Sender 12, beispielsweise eine Leuchtdiode, einen optischen Empfänger 13, beispielsweise eine Fotodiode, eine Kopplungseinheit 14, beispielsweise einen optischen Y-Koppler, und eine Detektionseinheit 15. Die Steuereinheit 6 kann beispielsweise einen Mikroprozessor und auf dem Mikroprozessor ablauffähige Softwaremodule zur Erstellung der Funktionen der Steuereinheit umfassen. Die Steuereinheit 6 kann Analog-Digital-Wandler, Digital-Analog-Wandler, elektronische Verstärker, Filter, Integratoren oder irgendwelche andere elektronischen Schaltungen zur Erstellung der Funktionen der Steuereinheit umfassen. Mittels des optischen Senders 12 werden über die Kopplungseinheit optische Signale in die optische Verbindung 3 eingekoppelt und zum optischen Sender geleitet. Wie erwähnt, werden diese optischen Signale je nach umgebendem Medium ganz oder teilweise reflektiert. Die reflektierten optischen Signale werden über die optische Verbindung 3 und über die Kopplungseinheit auf einen optischen Empfänger 13 geführt. Mittels der Detektionseinheit 15 werden die am optischen Empfänger 13 empfangbaren optischen Signale ausgewertet, beispielsweise wird die Energie von empfangenen optischen Signalen bestimmt. Überschreitet diese Energie einen gewissen Schwellwert, dann wird dies durch die Detektionseinheit 15 dem Ereignis zugeordnet, dass sich die Saugerspitze 2 in Luft befindet (mehr oder weniger vollständige Reflexion von optischen Signalen am optischen Sensor). Unterschreitet diese Energie einen gewissen Schwellwert, dann wird dies durch die Detektionseinheit 15 dem Ereignis zugeordnet, dass sich die Saugerspitze 2 in einem flüssigen Medium befindet. Je nachdem, ob sich der optische Sensor in Luft oder in einer Flüssigkeit befindet, wird durch die Detektionseinheit 15 ein entsprechendes Stellsignal zur Einstellung eines Schiebers der Schliesseinheit 7 erzeugt. In Figur 2 ist ein Schieber schematisch mit in zwei Richtungen zeigenden Pfeilen gezeichnet. Der Schieber der Schliesseinheit 7 kann beispielsweise im Wesentlichen ein Metallquader umfassen, welches Metallquader in einer Führung angeordnet ist und entsprechend dem durch die Detektionseinheit 15 erzeugte Stellsignal über eine Antriebseinheit zwischen zwei Positionen eingestellt wird. Die Antriebseinheit kann einen Elektromotor mit oder ohne Getriebe umfassen. Eine Schliesseinheit kann aus einem Metallquader und einem Metallkeil, welcher durch ein Motor und ein Getriebe angetrieben wird, bestehen. Eine Schliesseinheit kann auch aus einem Metallexzenter, welcher durch ein Motor und allenfalls ein Getriebe angetrieben wird, bestehen. In einer anderen Ausführungsvariante kann die Antriebseinheit einen Magnetantrieb umfassen, welcher durch ein starkes Magnetfeld ein Magnetventil ein- und ausschaltet. Mittels des Schiebers der Schliesseinheit 7 wird die Absaugleitung 8 zusammengedrückt und verschlossen, falls durch die Steuereinheit 6 detektiert wird, dass sich die Saugerspitze 2 in der Luft befindet. Der Schieber der Schliesseinheit 7 wird jedoch geöffnet, falls mittels der Steuereinheit 6 detektiert wird, dass sich die Saugerspitze 2 in einer Flüssigkeit befindet.

Mit einer solchen Absaugvorrichtung wird der Anteil der abgesaugten Luft minimiert. Zudem ist eine solche Absaugvorrichtung einfach in bestehende Infrastrukturen eines Spitals integrierbar. Dadurch, dass sich die Absaugleitung 8 mittels eines Quetschens in einer Quetscheinheit leicht wiederentfernbar in die Schliesseinheit aufgenommen werden kann, wird gewährleistet, dass bei einem Defekt der Schliesseinheit 7 oder der Steuereinheit 5 die Absaugvorrichtung rasch in eine Absaugvorrichtung gemäss dem Stand der Technik übergeführt werden kann und somit mit einer bekannten Absaugvorrichtung weitergearbeitet werden kann.

Selbstverständlich wird die im Reservoir 9 gesammelte Flüssigkeit, insbesondere Blut, welches durch den Sauger 1 abgesaugt wurde, über eine in Figur 1 gestrichelt gezeichnete Abführleitung aus dem Reservoir 9 abgeführt und für eine Weiterverwendung, also insbesondere für das Zurückführen von bei einer Operation anfallendem Blut in den Körper des Patienten, zur Verfügung gestellt. Beispielsweise kann so abgeführtes Blut in eine Herz-Lungen-Maschine geführt werden und dort dem Blutkreislauf des Patienten zurückgeführt werden. Im Stand der Technik sind Methoden bekannt, welche eine solche Rückführung bewerkstelligen.

## Patentansprüche

1. Absaugvorrichtung für das Absaugen von Flüssigkeiten in einem Operationsgebiet, mit einem Sauger (1), wobei an einer Saugerspitze (2) des Saugers (1) ein optischer Sensor (4) angebracht ist und über eine optische Verbindung (3) mit einer Steuereinheit (6) verbunden ist, wobei durch die Steuereinheit (6) und mittels des optischen Sensors (4) detektierbar ist, ob sich die Saugerspitze (2) in der Flüssigkeit oder in der Luft befindet, **dadurch gekennzeichnet, dass** der Sauger (1) über eine Absaugleitung (8) mit einem Reservoir (9) verbunden ist, dass das Reservoir (9) über eine Verbindungsleitung (10) mit einer Unterdruckeinheit (11) verbunden ist, dass an der Absaugleitung (8) eine durch die Steuereinheit (6) steuerbare Schliesseinheit (7) angebracht ist, mit welcher Schliesseinheit (7) die Absaugleitung (8) verschliessbar ist, falls mittels der Steuereinheit (6) detektierbar ist, dass sich die Saugerspitze (2) in der Luft befindet.

2. Absaugvorrichtung nach Anspruch 1, **dadurch gekennzeichnet, dass** die optische Verbindung (3) durch mindestens eine optische Lichtleiterfaser ausgeführt ist und dass der optische Sensor (4) durch einen Abschnitt der mindestens einen Lichtleiterfaser gebildet wird.

3. Absaugvorrichtung nach einem der Ansprüche 1 bis 2, **dadurch gekennzeichnet, dass** die Steuereinheit (6) einen optischen Sender (12) und einen optischen Empfänger (13) umfasst, wobei mittels einer Kopplungseinheit (14) optische Signale vom optischen Sender in die optische Verbindung (3) eingekoppelt und zum optischen Sensor (4) geführt werden, wobei der optische Sensor (4) derart ausgebildet ist, dass mit Luft respektive Flüssigkeit als umgebendes Medium das Licht total reflektiert respektive nur teilweise reflektiert wird und über die optische Verbindung (3) und die Kopplungseinheit (14) an den optischen Empfänger (13) zurückgeleitet wird, und dass die Steuereinheit eine Detektionseinheit (15) umfasst, welche derart ausgebildet ist, dass mittels einer Messung der durch den optischen Empfänger empfangbaren Lichtenergie detektiert wird, ob sich der optische Sensor in der Luft als umgebendes Medium oder in Flüssigkeit als umgebendes Medium befindet.

4. Absaugvorrichtung nach ein der Ansprüche 1 bis 3, **dadurch gekennzeichnet, dass** die Absaugleitung (8) aus einem deformierbaren Material besteht, dass die Schliesseinheit (7) eine Halterung zur Aufnahme der Absaugleitung (8) und einen Schieber umfasst, wobei der Schieber so angeordnet ist, dass die Absaugleitung (8) durch den Schieber deformierbar und vollständig verschliessbar ist.

5. Absaugvorrichtung nach Anspruch 4, **dadurch gekennzeichnet, dass** der Schieber mittels einer von einem Elektromotor ableitbaren Kraft antreibbar ist.

6. Absaugvorrichtung nach Anspruch 4, **dadurch gekennzeichnet, dass** der Schieber mittels einer von einem Magnetantrieb ableitbaren Kraft antreibbar ist.

7. Absaugvorrichtung nach Anspruch 4, **dadurch gekennzeichnet, dass** die Halterung zur Aufnahme der Absaugleitung (8) mindestens eine Quetscheinheit umfasst, wobei die Absaugleitung (8) in der Halterung durch ein Quetschen der Absaugleitung (8) in der Quetscheinheit wiederentfernbar anbringbar ist.

8. Absaugvorrichtung nach einem der Ansprüche 1 bis 7, **dadurch gekennzeichnet, dass** die Steuereinheit (6) ein Kalibriermodul umfasst, wobei beim Einschalten der Steuereinheit (6) mittels des optischen Senders (12) ein Lichtsignal in die optische Verbindung (3) eingekoppelt wird, wobei mittels des optischen Empfängers (13) die reflektierte Lichtenergie gemessen wird und wobei mittels des Kalibriermoduls die Schliessvorrichtung (7) für die gemessene Lichtenergie derart eingestellt wird, dass die Absaugleitung verschlossen ist.

## Claims

1. Suction device for removal of liquids in a surgical field, with an aspirator (1), an optical sensor (4) being installed on an aspirator tip (2) of the aspirator (1) and being connected to a control unit (6) via an optical connection (3), it being detectable by the control unit (6) and by means of the optical sensor (4) whether the aspirator tip (2) is located in the liquid or in the air, **characterised in that** the aspirator (1) is connected to a reservoir (9) via a suction line (8), **in that** the reservoir (9) is connected to a vacuum unit (11) via a connecting line (10), **in that** a closing unit (7), controllable by the control unit (6), is installed on the suction line (8), with which closing unit (7) the suction line (8) is closable if it is detectable by means of the control unit (6) that the aspirator tip (2) is located in the air.

2. Suction device according to claim 1, **characterised in that** the optical connection (3) is implemented through at least one optical fibre, and **in that** the optical sensor (4) is formed by a section of the at least one optical fibre.

3. Suction device according to one of the claims 1 to 2, **characterised in that** the control unit (6) comprises an optical transmitter (12) and an optical receiver (13), by means of a coupling unit (14) optical signals being launched from the optical transmitter into the optical connection (3) and led to the optical sensor (4), the optical sensor (4) being designed such that with air or respectively liquid as the surrounding medium the light is totally reflected or respectively only partially reflected and, via the optical connection (3) and the coupling unit (14), is led back to the optical receiver (13), and **in that** the control unit comprises a detection unit (15) which is designed such that, by means of a measurement of the light energy receivable by the optical receiver, detected is whether the optical sensor is located in the air as the surrounding medium or in liquid as the surrounding medium.

4. Suction device according to one of the claims 1 to 3, **characterised in that** the suction line (8) consists of a deformable material, **in that** the closing unit (7) comprises a holder for receiving the suction line (8) and a slide, the slide being disposed such that the suction line (8) is deformable by the slide and completely closable.

5. Suction device according to claim 4, **characterised in that** the slide is able to be powered by means of a force able to be diverted from an electromotor.

6. Suction device according to claim 4, **characterised in that** the slide is able to be powered by means of a force able to be diverted from a magnetic drive.

7. Suction device according to claim 4, **characterised in that** the holder for receiving the suction line (8) comprises at least one squeezing unit, the suction line (8) in the holder being removably placeable in the squeezing unit through a squeezing of the suction line (8).

8. Suction device according to one of the claims 1 to 7, **characterised in that** the control unit (6) comprises a calibrating module, upon switching on of the control unit (6), a light signal being launched in the optical connection (3) by means of the optical transmitter (12), the reflected light energy being measured by means of the optical receiver (13), and the closing device (7) being set for the measured light energy by means of the calibrating module such that the suction line is closed.

## Revendications

1. Dispositif pour l'aspiration de liquides dans un champ opératoire, avec un aspirateur (1), un capteur optique (4) étant placé à une pointe d'aspirateur (2) de l'aspirateur (1) et étant relié par une connexion optique (3) à une unité de commande (6), auquel cas l'unité de commande (6) et le capteur optique (4) peut détecter si la pointe d'aspirateur (2) se trouve dans l'air ou dans le liquide, **caractérisé en ce que** l'aspirateur (1) est relié à un réservoir (9) par une conduite d'aspiration (8), **en ce que** le réservoir (9) est relié à une unité de dépression (11) par une conduite de liaison (10), **en ce que** sur la conduite d'aspiration (8), il est ménagé une unité de fermeture (7) (7) commandable par l'unité de commande (6), unité de fermeture (7) avec laquelle la conduite d'aspiration (8) peut être fermée et l'unité de commande (6) peut détecter si la pointe d'aspirateur (2) se trouve dans l'air.

2. Dispositif d'aspiration selon la revendication 1, **caractérisé en ce que** la connexion optique (3) est réalisée par au moins une fibre optique et **en ce que** le capteur optique (4) est formé par une section d'au moins une fibre optique.

3. Dispositif d'aspiration selon l'une des revendications 1 à 2, **caractérisé en ce que** l'unité de commande (6) comprend un capteur optique (12) et un récepteur optique (13), des signaux optiques du capteur optique étant accouplés au moyen d'une unité d'accouplement (14) dans la liaison optique (3) et étant amenés au capteur optique (4), le capteur optique (4) étant réalisé de sorte qu'avec l'air respectivement le liquide en tant que milieu environnant, la lumière est réfléchie entièrement respectivement partiellement et est ramené par la liaison optique (3) et l'unité d'accouplement (14) au récepteur optique (13) et **en ce que** l'unité de commande comprend une unité de détection (15) qui est conçue de sorte qu'au moyen d'une mesure de l'énergie lumineuse recevable par le récepteur optique si le capteur optique se trouve dans l'air en tant que milieu environnant ou dans le liquide en tant que milieu environnant.

4. Dispositif d'aspiration selon l'une des revendications 1 à 3, **caractérisé en ce que** la conduite d'aspiration (8) se compose de matériau déformable, **en ce que** l'unité de fermeture (7) comprend une fixation pour le logement de la conduite d'aspiration (8) et un curseur, le curseur étant disposé de sorte que la conduite d"aspiration (8) peut être déformée et complètement fermée par le curseur.

5. Dispositif d'aspiration selon la revendication 4, **caractérisé en ce que** le curseur peut être entraîné au moyen d'une force dérivée d'un moteur électrique.

6. Dispositif d'aspiration selon la revendication 4, **caractérisé en ce que** le curseur peut être entraîné au moyen d'une force dérivée d'un entraînement magnétique.

7. Dispositif d'aspiration selon la revendication 4, **caractérisé en ce que** la fixation pour le logement de la conduite d'aspiration (8) comprend au moins une unité de compression, la conduite d'aspiration (8) étant placée dans la fixation par une compression de la conduite d'aspiration (8) dans l'unité de compression de manière amovible.

8. Dispositif d'aspiration selon l'un des revendications 1 à 7, **caractérisé en ce que** l'unité de commande (6) comprend un module de calibrage, lors de la mise en route de l'unité de commande (6) au moyen du capteur optique (12), un signal lumineux étant accouplé dans la liaison optique (3), l'énergie lumineuse réfléchie étant mesurée au moyen du récepteur optique (13) et le dispositif de fermeture (7) pour l'énergie lumineuse mesurée étant réglé au moyen du module de calibrage de sorte que la conduite d'aspiration est fermée.
